# EUROPÄISCHE PATENTSCHRIFT

(11) **EP 0 445 642 B1**
(45) Veröffentlichungstag und Bekanntmachung des Hinweises auf die Patenterteilung: **12.07.1995**
(21) Anmeldenummer: 91102906.4
(22) Anmeldetag: 28.02.1991
(51) Int. Cl.: C07D 239/46, C07D 239/52, C07D 239/34, C07D 239/60

(54) **2-Amino-(fluoralkoxy-pyrimidine) und Verfahren zu ihrer Herstellung**
2-Amino-(fluoralkoxy-pyrimidines) and process for their preparation
2-Amino-(fluoralkoxy-pyrimidines) et procédé pour leur préparation

(30) Priorität: 08.03.1990 DE 4007316
(43) Veröffentlichungstag der Anmeldung: 11.09.1991
(73) Patentinhaber: BASF Aktiengesellschaft, 67063 Ludwigshafen (DE)
(72) Erfinder: Hamprecht, Gerhard, Dr., W-6940 Weinheim (DE)

(56) Entgegenhaltungen:
- EP-A- 0 158 594
- EP-A- 0 169 815
- EP-A- 0 271 833
- EP-A- 0 378 089

## Beschreibung

Die Erfindung betrifft neue 2-Amino-(fluoralkoxy-pyrimidine) der allgemeinen Formel I, 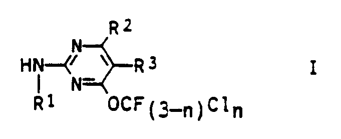
in der
- R¹: Wasserstoff, C₁-C₄-Alkyl, C₃-C₄-Alkenyl oder C₃-C₄-Alkinyl
- R²: Wasserstoff, Halogen oder C₁-C₄-Halogenalkyl sowie auch einen Trifluor- bzw. Chlordifluormethoxyrest
- R³: Wasserstoff
- n: 0 oder 1
bedeuten. Weiterhin betrifft die Erfindung ein Verfahren zur Herstellung der Pyrimidine I durch Umsetzung von Fluoralkoxy-2-halogen-pyrimidinen mit Aminen.

Die Pyrimidine I sind wertvolle Zwischenprodukte für organische Synthesen, insbesondere für die Herstellung von Pflanzenschutzmitteln.

In der Literatur finden sich vielfältige Hinweise, die aufgrund ähnlicher elektronischer Eigenschaften Fluoralkyl- und Fluoralkoxygruppen mit denen der Halogene gleichsetzen. pKa-Messungen (Proc. Acad. Sci. 134, 1207 (1960), J. Am. Chem. Soc. 83, 4860, (1961)) belegen, daß beispielsweise Fluoralkoxygruppen induktiv Elektronen ziehen, umgekehrt aufgrund ihrer Resonanzfähigkeit aber auch als Elektronendonor fungieren. Über alle Effekte gemessen ist die Trifluormethoxygruppe sogar stärker desaktivierend als die Halogene, so daß man auch von "Super-Halogenen" spricht (J. Am. Chem. Soc. 83, 4860, 1961). Dies gilt gleichermaßen für ihre Substituierbarkeit durch Nucleophile. Chemical Abstracts 87, 53396 belegt beispielsweise, daß 2,4-Bis(trichlormethyl)-6-trifluormethyl-1,3,5-triazin beim Rühren in Benzol mit basischen Aminen zwei Halogenalkylgruppen austauscht. Die Fähigkeit des Trifluormethoxyrestes als Austrittsgruppe zu fungieren wird beispielsweise auch in der Zuckerchemie benutzt (CA. 105, 115325; 107, 96978).

Vor diesem Hintergrund sind daher die bestehenden Verfahren zur Herstellung von 2-Amino-(fluoralkoxy-pyrimidinen) sehr umständlich und aufwendig. Beispielsweise muß ein entsprechend 2-Halogen-substituiertes Pyrimidin zunächst mit dem giftigen Methylmercaptan in die 2-Methyl-thio-pyrimidinverbindung umgewandelt, dann zum 2-Methylsulfonylderivat oxidiert und dann mit Amin nucleophil wieder verdrängt werden (US-A 4 542 216).

Aus der EP-A 271 833 ist ein Verfahren zur Herstellung von 4,6-disubstituierten 2-Aminopyrimidinen bekannt, bei dem ein Propandiimidat mit einem Cyansäureester umgesetzt werden.

Die EP-A 169 815 beschreibt N-Arylsulfonyl-N'-pyrimidinolharnstoffe, bei deren Herstellung 4-Chlordifluoromethoxy-2-aminopyrimidinderivate als Zwischenprodukte verwendet werden. Die 4-Chlordifluoromethoxy-2- aminopyrimidinderivate werden durch Umsetzung von 2-Amino-4-hydroxypyrimidinderivaten mit Difluorbromchlormethan oder Difluordibrommethan in Gegenwart einer Base hergestellt.

Demgemäß wurde gefunden, daß man die neuen 2-Amino-(fluoralkoxy-pyrimidine) der allgemeinen Formel I, 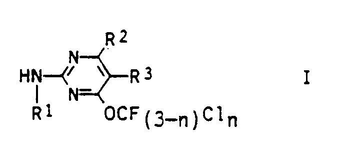
in der
R¹, R², R³ und n die vorgenannte Bedeutung besitzen vorteilhaft erhält, wenn man 2-Halogen-pyrimidine der Formel II, 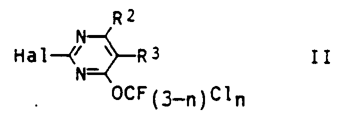
in der Hal für Fluor oder Chlor steht und R², R³ und n die vorgenannte Bedeutung besitzen, mit einem Amin der Formel III,

H-NH-R¹ III

in der R¹ die vorgenannte Bedeutung hat, umsetzt.

Die Umsetzung kann für den Fall der Verwendung von 2,4-Difluor-6-trifluormethoxy-pyrimidin und Ammoniak durch folgendes Schema beschrieben werden: 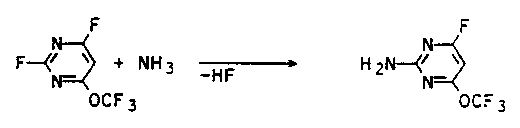
Das Verfahren liefert auf einfachem und wirtschaftlichem Weg neue 2-Amino-(fluoralkoxy-pyrimidine) in hoher Ausbeute und Reinheit. Entgegen der Erwartung werden Fluoralkoxygruppen unter den Reaktionsbedingungen nicht substituiert. Im Hinblick auf den Stand der Technik sind alle diese vorteilhaften Eigenschaften überraschend.

Bevorzugte Endstoffe I und dementsprechend bevorzugte Ausgangsstoffe II sind solche, in deren Formeln R¹ Wasserstoff, C₁-C₄-Alkyl wie Methyl, Ethyl, n-Propyl, i-Propyl, n-Butyl, sek-Butyl, i-Butyl, tert.-Butyl; C₃-C₄-Alkenyl wie 2-Propenyl, 2-Methylethenyl, 2-Butenyl, 3-Butenyl, 1-Methyl-2-propenyl oder 2-Methyl-2-propenyl; C₃-C₄-Alkinyl wie Propargyl, 2-Butinyl, 3-Butinyl oder 1-Methyl-2-propinyl, R² und R³ unabhängig voneinander Wasserstoff, Fluor, Chlor, Brom, Trichlormethyl, Dichlorfluormethyl, Chlordifluormethyl, Trifluormethyl, 1,1-Dichlor-2, 2,2-trifluorethyl, 1,1,2,2,2-Pentafluorethyl oder 1,1,2,2,2-Pentachlorethyl, darüberhinaus R² auch einen Trifluor- bzw. Chlordifluormethoxyrest und n 0 oder 1 bedeuten.

Unter den Aminen, welche eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin.

Die 2-Halogen-pyrimidine II können in einem aprotisch polaren Lösungsmittel mit den Aminen III bei einer Temperatur von -80 bis 40^{o}C umgesetzt werden, wobei man das Amin III entweder in einem Überschuß, bezogen auf II, einsetzt oder eine organische Hilfsbase verwendet.

Die Umsetzung des 2-Halogen-pyrimidins II mit dem Amin III kann in Abwesenheit oder vorteilhaft in Gegenwart eines Lösungsmittels vorgenommen werden. Als Lösungsmittel sind insbesondere die nachfolgend aufgeführten geeignet:
Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid, 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff II.

Vorteilhaft gibt man 1,8 bis 2,5, insbesondere 1,95 bis 2,2 Molequivalent des Amins III, bezogen auf den Ausgangsstoff II innerhalb 0,5 bis 2 Stunden zu einer Mischung von Ausgangsstoff II in einem der vorgenannten Lösungsmittel bei (-80) bis 40°C, Vorzugsweise -70 bis 25°C, rührt bis zur Vervollständigung der Reaktion nach (ca. 3 Stunden) und läßt dann zur Aufarbeitung auf 25°C erwärmen.

Setzt man nur ungefähr stöchiometrische Mengen des Amins III ein, so verwendet man zweckmäßig zusätzlich eine organische Hilfsbase, um den entstehenden Halogenwasserstoff abzufangen. Als Hilfsbase eignen sich dazu übliche organische Basen wie Trimethylamin, Triethylamin, N-Ethyl-diisopropylamin, Triisopropylamin, N,N-Dimethylanilin, N,N-Dimethylcyclohexylamin, N-Methylpyrrolidln, Pyridin, Chinolin, α-,β-,γ-Picolin, 2,4- und 2,6-Lutidin und Triethylendiamin. Im allgemeinen sind Zusätze von 0,9 bis 1,1 Equivalenten der Hilfsbase, bezogen auf den Ausgangsstoff II, ausreichend.

Die Reaktion kann drucklos oder unter Druck, kontinuierlich oder diskontinuierlich durchgeführt werden.

Die Aufarbeitung kann in üblicher Weise erfolgen, z.B. extrahiert man das Umsetzungsgemisch mit Wasser zur Entfernung der Salze, trocknet und reinigt die organische Phase, z. B. durch Chromatographie. Man kann jedoch auch direkt die organische Phase einengen und den Rückstand mit einem Lösungsmittel verrühren.

Im Hinblick auf ihre Weiterverarbeitung zu herbiziden Wirkstoffen, z.B. Sulfonylharnstoffderivaten, sind folgende Pyrimidine der Formel I besonders bevorzugt:
2-Amino-4-chlor-6-trifluormethoxy-pyrimidin,
2-Amino-4-fluor-6-trifluormethoy-pyrimidin,
2-Amino-4-chlor-6-chlordifluormethoxy-pyrimidin,
2-Amino-4-fluor-6-chlordifluormethoxy-pyrimidin,
2-Amino-4,6-bis-trifluormethoxy-pyrimidin,
2-Amino-4,6-bis-chlordifluormethoxy-pyrimidin,
2-Amino-4-trifluormethoxy-6-trifluormethyl-pyrimidin,
2-Amino-4-chlordifluormethoxy-6-trifluormethyl-pyrimidin,
2-Methylamino-4-chlor-6-trifluormethoxy-pyrimidin,
2-Methylamino-4-fluor-6-trifluormethoy-pyrimidin,
2-Methylamino-4-chlor-6-chlordifluormethoxy-pyrimidin,
2-Methylemino-4-fluor-6-chlordifluormethoxy-pyrimidin,
2-Methylamino-4,6-bis-trifluormethoxy-pyrimidin,
2-Methylamino-4,6-bis-chlordifluormethoxy-pyrimidin,
2-Methylamino-4-trifluormethoxy-6-trifluormethyl-pyrimidin,
2-Methylamino-4-chlordifluormethoxy-6-trifluormethyl-pyrimidin,
2-Allylamino-4-chlor-6-trifluormethoxy-pyrimidin,
2-Allylamino-4-fluor-6-trifluormethoy-pyrimidin,
2-Allylamino-4-chlor-6-chlordifluormethoxy-pyrimidin,
2-Allylamino-4-fluor-6-chlordifluormethoxy-pyrimidin,
2-Allylamino-4,6-bis-trifluormethoxy-pyrimidin,
2-Allylamino-4,6-bis-chlordifluormethoxy-pyrimidin,
2-Allylamino-4-trifluormethoxy-6-trifluormethyl-pyrimidin,
2-Allylamino-4-chlordifluormethoxy-6-trifluormethyl-pyrimidin,
Der Herstellung der als Ausgangsstoffe II benötigten 2-Halogenpyrimidine erfolgt nach dem in der zeitgleichen deutschen Anmeldung P 40 07 317.3 (O.Z. 0050/41450) beschriebenen Verfahren, das durch folgendes Reaktionsschema dargestellt wird: 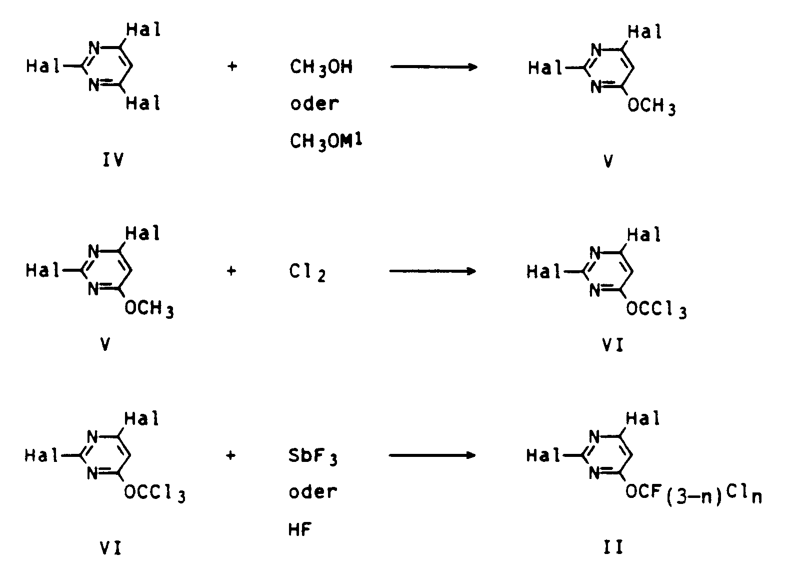
So kann man beispielsweise ein 2,4,6-Trihalogenpyrimidin IV in einem aprotisch polaren Lösungsmittel
a) mit Methanol in Anwesenheit oder Abwesenheit einer Base oder
b) mit einem Methanolat, wobei M¹ ein Alkalimetallkation wie Lithium-, Natrium- und Kaliumkation oder das Equivalent eines Erdalkalimetallkations wie Magnesium-, Calcium- und Bariumkation bedeutet, in Gegenwart von Methanol
bei Temperaturen zwischen -40 und 120^{o}C zum Methoxy-pyrimidin umsetzen. Diese Reaktionen können drucklos oder unter Druck (1 bis 10 bar, vorzugsweise 1 bis 5 bar), kontinuierlich oder diskontinuierlich durchgeführt werden.

Hal im Reaktionsschema steht für Fluor, Chlor oder Brom.

Für die Umsetzung des Trihalogenpyrimidins mit Methanol sind folgende Lösungsmittel geeignet:
Ether wie Methyl-tert.-butylether, Diethylether, Ethylpropylether, n-Butylethylether, Di-n-butylether, Diisobutylether, Diisoamylether, Di-isoopropylether, Cyclohexylmethylether, Tetrahydrofuran, 1,2-Dimethoxyethan, Diethylenglycoldimethylether und Anisol, chlorierte Kohlenwasserstoffe wie 1,1,2,2-Tetrachlorethan, 1,1-Dichlorethylen, Chlorbenzol, 1,2-Dichlorbenzol und 1-Chlornaphthalin sowie entsprechende Gemische.

Zweckmäßig verwendet man das Lösungsmittel in einer Menge von 100 bis 2000 Gew.-% vorzugsweise 500 bis 1500 Gew.%, bezogen auf den Ausgangsstoff.

Zweckmäßig führt man jedoch die Umsetzung der Ausgangsstoffe IV direkt in überschüssigem Methanol als Lösungsmittel durch. Gegebenenfalls gibt man ein Alkalimethanolat in einer equivalenten Menge oder einem Über- oder Unterschuß von bis zu 5 Mol-%, bezogen auf den Ausgangsstoff, zu einer Suspension des Ausgangsstoffes in der 5- bis 20-fachen Gewichtsmenge Methanol als Lösungsmittel, bezogen auf den Ausgangsstoff IV, innerhalb bis zu einer Stunde bei einer Temperatur von ca. -20 bis 80^{o}C. Zur Beendigung der Umsetzung rührt man dann noch ca. 1/2 bis 8 Stunden bei 0 bis 120^{o}C, vorzugsweise 0 bis 100^{o}C nach.

Zur Isolierung der Methoxy-pyrimidine dienen die hierfür in der Literatur üblichen Aufarbeitungsmethoden.

Die Chlorierung des Methoxy-pyrimidins mit Chlor zu dem Trichlormethoxypyrimidin führt man beispielsweise bei einer Temperatur von 60 bis 180^{o}C durch.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Braunstein oder durch anodische Chlorierung hergestellt werden.

Die Chlorierung kann in Gegenwart eines inerten Lösungsmittels, beispielsweise einem chlorierten Kohlenwasserstoff wie Chloroform, Tetrachlorkohlenstoff, Chlorbenzol, 1,2-, 1,3- oder 1,4-Dichlorbenzol, einem Nitril wie Acetonitril, Propionitril, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von α,α'-Azoisobutyronitril, zweckmäßig in einer Menge von 0,2 bis 7 Mol-%, bezogen auf den Ausgangsstoff. Man kann die Reaktion auch durch Zugabe eines Katalysators beschleunigen; als solcher eignet sich Phosphorpentachlorid, zweckmäßig in einer Menge von 0,5 bis 7 Mol-% bezogen auf den Ausgangsstoff V. In diesem Fall legt man den Ausgangsstoff V zusammen mit dem Katalysator vor und beginnt dann mit der Chlorierung. Statt des Phosphorpentachlorids kann man auch eine dieses unter den Reaktionsbedingungen bildende Ausgangskomponente, z. B. Phosphortrichlorid oder gelben Phosphor, zugeben und dann mit der Chlorierung beginnen.

Ausgangsstoff V kann mit Chlor in annähernd stöchiometrischer Menge oder vorzugsweise im Überschuß, vorteilhaft mit 3,1 bis 11, insbesondere 3,3 bis 5 Mol Chlor je Equivalent Methoxy in dem Ausgangsstoff V umgesetzt werden. Die Umsetzung kann bei einer Temperatur von 60 bis 180^{o}C, vorteilhaft von 100 bis 150^{o}C, drucklos oder unter Druck kontinuierlich oder diskontinuierlich durchgeführt werden.

Chloriert man bei 1 bar, so werden zweckmäßig 3,3 bis 5 Mol Chlorgas, bezogen auf ein Equivalent Methoxy in dem Ausgangsstoff V, eingesetzt, was einem Chlorumsatz von 91 bis 60 % entspricht. Durch geeignete apparative Maßnahmen, z. B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in den flüssigen Ausgangsstoff V einleitet, wobei man zunächst bei einer Temperatur von 60 bis 80^{o}C beginnt und diese - gegebenenfalls unter Ausnutzung des exothermen Charakters der Reaktion - kontinuierlich steigert, so daß gegen Ende die Umsetzung bei einer Temperatur von 100 bis 150^{o}C durchgeführt wird. Bei größeren Reaktionsansätzen muß dem exothermen Charakter durch äußere Kühlung bzw. geeignete Dosierung der Chlormenge Rechnung getragen; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung des Trichlormethoxy-pyrimidins VI mit einem Halogenaustauschmittel führt man beispielsweise bei einer Temperatur von 0 bis 170^{o}C durch.

Als Halogenaustauschmittel sind Antimontrifluorid in Gegenwart oder Abwesenheit katalytischer Mengen eines Antimon(V)salzes, z.B. Antimon(V)chlorid oder Fluorwasserstoff geeignet.

Zweckmäßig verwendet man einen Überschuß von 1 bis 200, vorzugsweise 5 bis 20 Mol-% Antimontrifluorid pro Trichlormethylequivalent. Die Katalysatormenge an Antimon(V)salz beträgt zwischen 1 bis 20, vorzugsweise 5 bis 18 Mol-% pro Trichlormethylequivalent. Vorzugsweise dosiert man den Ausgangsstoff VI bei 90 bis 130^{o}C zu der Mischung des Halogenaustauschmittels und erwärmt dann noch zwischen 10 und ca. 120 Minuten auf eine Temperatur zwischen 140 bis 170^{o}C. Anschließend wird durch Destillation aufgearbeitet.

Man kann die Reaktion jedoch auch kontinuierlich führen, den Ausgangsstoff VI bei 140 bis 170°C innerhalb 10 bis ca. 120 Minuten zugeben und gleichzeitig unter vermindertem Druck den entstehenden niedriger siedenden Endstoff II abdestillieren. Spuren mitgerissender Antimonsalze lassen sich durch Extraktion mit konz. Salzsäure beseitigen.

Arbeitet man ohne Antimon(V)salz-Katalyse oder setzt nur geringe Mengen, z. B. 0,2 bis 1 Mol-% ein, und reduziert die Menge an Antimontrifluorid auf 60 bis 90 Mol-% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 170^{o}C, vorzugsweise 40 bis 120^{o}C, durchgeführt werden. Hierzu versetzt man in einem Autoklaven den Ausgangsstoff VI mit einem Überschuß von 300 bis 700, vorzugsweise 350 bis 400 Mol-% Fluorwasserstoff pro Trichlormethylequivalent und rührt 10 Minuten bis ca. 10 Stunden nach. Im allgemeinen ist eine Nachreaktionszeit von ca. 4 Stunden ausreichend. Nach dem Entspannen und der Entfernung flüchtiger Bestandteile wird wie beschrieben aufgearbeitet.

Die neuen 2-Amino-(fluoralkoxy-pyrimidine) I sind wertvolle Zwischenprodukte für die Herstellung von Pflanzenschutzmitteln. Nach dem Verfahren der zeitgleichen deutschen Patentanmeldung P 40 07 683.0 (O.Z. 0050/41452) können sie durch Umsetzung von beispielsweise 2-Amino-4-fluor-6-trifluormethoxy-pyrimidin mit Methanol in das entsprechende 2-Amino-6-methoxy-4-trifluormethoxy-pyrimidin umgewandelt werden, das mit 2-Carbomethoxy-benzolsulfonylisocyanat zu herbiziden Sulfonylharnstoffen reagiert. Sie können aber auch direkt mit dem genannten Isocyanat zu herbiziden Sulfonylharnstoffen umgesetzt werden.

### Beispiele

### I Herstellbeispiele für die Vorprodukte (vgl. die zeitgleiche deutsche Anmeldung P 40 07 317.3 (O.Z. 0050/41450)

### Beispiel I.1 2-Chlor-4-trichlormethoxy-6-trichlormethylpyrimidin

### a) 2-Chlor-4-methoxy-6-trichlormethylpyrimidin

293,1 g (1,692 mol) 3%ige Natriummethylatlösung wurden innerhalb 1 1/2 Stunden bei 0 bis 5^{o}C unter Rühren zu einer Lösung von 434 g (1,692 mol) 2,6-Dichlor-4-trichlormethylpyrimidin in 1 l 1,2-Dichlorethan gegeben. Es wurde 1 Stunde bei 0 bis 5^{o}C und 12 Stunden bei 25^{o}C gerührt. Das Reaktionsgemisch wurde mit Wasser und mit gesättigter Kochsalzlösung extrahiert. Nach dem Trocknen über Magnesiumsulfat und Einengen erhielt man 423 g (95 % d. Th.) der Titelverbindung als fast farbloses Öl. ¹H-NMR (CDCl₃) (ppm) OCH₃ (s/3H) 4,1; CH (s/1H) 7,25.

### b) 2-Chlor-4-trichlormethoxy-6-trichlormethylpyrimidin

In eine Mischung von 210 g (0,802 mol) a) und 260 mg (0,0016 mol), α,α'-Azoisobutyronitril wurden unter UV-Bestrahlung und gaschromatographischer Kontrolle des Reaktionsverlaufs bei einer Temperatur von zunächst 110^{o}C Chlor eingeleitet, wobei sich auch nach Entfernen der Heizbades eine Reaktionstemperatur von 140^{o}C einstellte. Nach dem Abklingen der Reaktion wurden während 5 1/2 Stunden bei 120^{o}C insgesamt 341 g (4,8 mol) Chlor eingeleitet. Zu dem erkaltenden Reaktionsgemisch rührte man ab 40^{o}C 70 ml n-Pentan zur Ausfällung hinzu. Der Niederschlag wurde abgesaugt, mit Petrolether gewaschen und getrocknet, wobei man 163 g (55 % d. Th.) der Titelverbindung vom Fp. 67-69^{o}C erhielt.

Das Filtrat (113,8 g) bestand nach dem Gaschromatogramm zu 83 % aus der Titelverbindung, 4 % 2-Chlor-4-dichlormethoxy-6-trichlormethylpyrimidin und 9 % 2,4-Dichlor-6-trichlormethylpyrimidin. Die Gesamtausbeute der Titelverbindung betrug 87,6 % d. Th.

### Beispiel I.2 2, 4-Difluor-6-trichlormethoxy-pyrimidin

### a) 2,4-Difluor-6-methoxypyrimidin (Herstellung nach dem Verfahren der älteren deutschen Patentanmeldung p 39 00 471.6)

Zu einer Mischung aus 250 g (1,865 mol) 2,4,6-Trifluorpyrimidin in 1,4 l Methanol wurden bei -20^{o}C innerhalb von 45 Minuten 335,8 g (1,865 mol) 30%iges Natriummethylat (in Methanol) gegeben und weitere 30 Minuten bei dieser Temperatur gerührt. Anschließend ließ man auf 25^{o}C erwärmen und engte die Reaktionsmischung auf ca. 1/5 ihres Volumens ein.

Das so erhaltene Gemisch wurde zwischen Diethylether und Wasser verteilt, wonach die organische Phase über Magnesiumsulfat getrocknet und eingeengt wurde. Nach Destillation (1,1 m Kolonne, 3 mm V-Füllkörper) erhielt man 141,6 g (52 % d. Th.) der Titelverbindung von Kp.: 144-145°C.

Aus dem Destillationsrückstand erhielt man durch Destillation über einen Normag-Aufsatz 114,4 g (42 % d. Th.) an 4,6-Difluor-2-methoxy-pyrimidin vom Kp.: 157-161^{o}C.

### b) 2,4-Difluor-6-trichlormethoxypyrimidin

210 g (2,96 mol) Chlor wurden unter UV-Bestrahlung und gaschromatographischer Kontrolle des Reaktionsverlaufs während 2 1/2 Stunden unter Rühren bei 130^{o}C in 123 g (0,843 mol) 2,4-Difluor-6-methoxy-pyrimidin eingeleitet. Das Reaktionsgemisch wurde über eine 10-cm-Vigreux-Kolonne im Vakuum destilliert, wobei man 190,2 g (90,5 % d. Th.) der Titelverbindung vom Sdp. 40-43^{o}C/0,2 mbar erhielt.

### Beispiel I.3 2,4-Dichlor-6-trichlormethoxy-pyrimidin

Unter Rühren, UV-Bestrahlung und gaschromatographischer Kontrolle des Reaktionsverlaufs wurden 303 g (4,27 mol) Chlor während 1/2 Stunde bei 80^{o}C, 1 Stunde bei 100^{o}C, 3 Stunden bei 120^{o}C und 3 Stunden bei 150^{o}C in eine Mischung von 209 g (1,168 Mol) 2,6-Dichlor-4-methoxy-pyrimidin und 2 g (0,012 mol) α,α'-Azoisobutyronitril eingeleitet. Anschließend wurde das Reaktionsgemisch im Vakuum über eine 50-cm-Kolonne mit 4 mm V₂-A-Raschigringen destilliert. Man erhielt 241,3 g (73 % d. Th.) der Titelverbindung vom Sdp. 87-88^{o}C/0,4 mbar; Fp. 55-56^{o}C.

### Beispiel I.4 2,4-Difluor-6-trifluormethoxy-pyrimidin

49,9 g (0,2 mol) 2,4-Difluor-6-trichlormethoxy-pyrimidin wurde bei 100^{o}C innerhalb 15 Minuten unter Rühren zu einer Mischung von 39,3 g (0,22 mol) Antimontrifluorid und 9,38 g (0,031 mol) Antimonpentachlorid gegeben. Innerhalb 25 Minuten wurde die Badtemperatur von 100 auf 150^{o}C erhöht und 30 Minuten nachgerührt, wobei sich zwischen 120 bis 125^{o}C Rückfluß einstellte. Durch anschließende Destillation erhielt man 37,1 g (92,7 % d. Th.) der Titelverbindung vom Sdp. 125-127^{o}C.

### Beispiel I.5 6-Chlordifluormethoxy-2,4-difluor-pyrimidin

93 g (0,373 mol) 2,4-Difluor-6-trichlormethoxy-pyrimidin wurden bei 100^{o}C innerhalb 10 Minuten unter Rühren zu einer Mischung von 44,5 g (0,249 mol) Antimontrifluorid und 0,94 g (0,0031 mol) Antimonpentachlorid gegeben. Innerhalb 25 Minuten wurde die Badtemperatur von 100 auf 175^{o}C erhöht, wobei sich bei 145^{o}C Rückfluß einstellte. Nach 1 1/2 Stunden Rühren wurde das Reaktionsprodukt bei 146-150^{o}C abdestilliert. Das Destillat wurde in 200 ml Methylenchlorid gelöst, mit 6 n Salzsäure extrahiert und über Magnesiumsulfat getrocknet. Nach dem Einengen im Vakuum erhielt man als Rückstand die Titelverbindung in einer Ausbeute von 63,7 g = 78,8 % d. Th.

### Beispiel I.6 2-Fluor-4-trifluormethoxy-6-trifluormethyl-pyrimidin

80 g (0,219 mol) 2-Chlor-4-trichlormethyl-6-trichlormethoxy-pyrimidin wurden innerhalb 5 Minuten unter Rühren bei 100^{o} zu einer Mischung von 93,9 g (0,525 mol) Antimontrifluorid und 18,7 g (0,0627 mol) Antimonpentachlorid gegeben. Innerhalb 10 Minuten wurde die Badtemperatur auf 140^{o}C erhöht und 1 Stunde nachgerührt, wobei sich starker Rückfluß einstellte. Das Reaktionsprodukt wurde bei 135-140^{o}C, gegen Schluß bei 95^{o}C/50 mbar, überdestilliert. Das Destillat wurde in Methylenchlorid aufgenommen, mit 6 n Salzsäure extrahiert und über Magnesiumsulfat getrocknet. Nach dem Einengen im Vakuum erhielt man die Titelverbindung in einer Ausbeute von 35,9 g (65,5 % d. Th.).

### Beispiel I.7 2,4-Dichlor-6-trifluormethoxy-pyrimidin

115 g (0,407 mol) 2,4-Dichlor-6-trichlormethoxy-pyrimidin wurden innerhalb 5 Minuten unter Rühren bei 100^{o}C zu einer Mischung von 80 g (0,447 mol) Antimontrifluorid und 18,77 g (0,0627 mol) Antimonpentachlorid gegeben, wobei sich die Reaktionstemperatur bis auf 140^{o}C erhöhte. Es wurde noch 45 Minuten bei 150^{o}C gerührt. Zur Destillation wurde ein Druck von 210 mbar eingestellt, wobei die Titelverbindung bei 128^{o}C überging; letzte flüchtige Bestandteile wurden bei 110^{o}C/22 mbar übergetrieben. Das Destillat wurde in Methylenchlorid gelöst, mit 6 n Salzsäure extrahiert und über Magnesiumsulfat getrocknet. Nach dem Einengen im Vakuum erhielt man die Titelverbindung in einer Ausbeute von 80 g (84,4 % d. Th.) als farbloses Öl vom n_{D}²⁵ = 1,4604.

### II Herstellung der erfindungsgemäßen Verbindungen I

### Beispiel II.1 2-Amino-4-chlordifluormethoxy-6-fluor-pyrimidin

9,8 g (0,578 mol) gasförmiges Ammoniak wurden innerhalb einer Stunde bei -75 bis -70^{o}C unter Rühren in eine Mischung von 62,5 g (0,289 mol) 2,4-Difluor-6-chlordifluormethoxy-pyrimidin in 300 ml Tetrahydrofuran eingegast. Es wurde eine Stunde bei -70^{o}C gerührt und dann auf Raumtemperatur erwärmt. Der ausgefallene Niederschlag wurde abgesaugt, zwischen Essigester und Wasser verteilt und die organische Phase über Magnesiumsulfat getrocknet. Das Reaktionsfiltrat wurde eingeengt, in der obigen Essigesterphase gelöst, über Kieselgel mit Petrolether:Ether = 5:1 chromatographiert und eingeengt. Man erhielt 46,5 g (75,3 % d. Th.) der Titelverbindung als farblose Kristalle vom Fp. 77-80^{o}C.

### Beispiel II.2 2-Amino-4-fluor-6-trifluormethoxy-pyrimidin

8,7 g (0,51 mol) gasförmiges Ammoniak wurden innerhalb 1 Stunde bei - 75 bis -70^{o}C unter Rühren in eine Mischung von 51 g (0,255 mol) 2,4-Difluor-6-trifluormethoxy-pyrimidin in 200 ml Diethylether eingegast. Es wurde noch 1 1/2 Stunden bei -70^{o}C und 1 Stunde bei Raumtemperatur gerührt. Das Reaktionsgemisch wurde im Vakuum eingeengt, in Methylenchlorid aufgenommen und mit Wasser extrahiert. Nach dem Trocknen der organischen Phase, Einengen und Chromatographieren über Kieselgel mit Petrolether:Ether = 8:1 erhielt man 38,1 g (75,6 % d. Th.) der Titelverbindung als farblose Kristalle vom Fp. 86-89^{o}C.

### Beispiel II.3 2-Amino-4-chlor-6-trifluormethoxy-pyrimidin

4,3 g (0,25 mol) gasförmiges Ammoniak wurden innerhalb 45 Minuten unter Rühren bei -50 bis -45°C in eine Mischung von 23,3 g (0,1 mol) 2,4-Di-chlor-6-trifluormethoxy-pyrimidin in 150 ml Methyl-tert.-butylether eingeleitet. Es wurde 30 Minuten bei -50^{o}C, 1 Stunde bei -30^{o}C und 1 Stunde bei 25^{o}C gerührt. Der ausgefallene Niederschlag wurde abgesaugt, mit Wasser gewaschen und getrocknet, wobei man 5,4 g (33,1 % d. Th.) 4-Amino-2,4-dichlorpyrimidin vom Fp. 270-272^{o}C als Nebenprodukt erhielt. Das Filtrat wurde mit Wasser gewaschen, getrocknet, teilweise im Vakuum eingeengt und mit Petrolether:Ether = 5:1 frakioniert chromatographiert, wobei man in den ersten Fraktionen 3 g (12,8 % d. Th.) Ausgangsmaterial als farbloses Öl und im Nachlauf 9 g (42 % d. Th.) der Titelverbindung als farblose Kristalle vom Fp. 55-56°C erhielt. Der Umsatz betrug 48,3 %.

### Beispiel II.4 4-Chlordifluormethoxy-6-fluor-2-methylamino-pyrimidin

20,3 g (0,0938 mol) 4-Chlordifluormethoxy-2,6-difluor-pyrimidin wurden in 150 ml Tetrahydrofuran vorgelegt und bei -70 bis -60^{o}C innerhalb 30 Minuten unter Rühren mit 5,8 g (0,188 mol) gasförmigem Methylamin versetzt. Es wurde jeweils 1 Stunde bei -70^{o}C, 0^{o}C und 25^{o}C gerührt. Nach dem Einengen des Reaktionsgemisches im Vakuum wurde der Rückstand mit Wasser verrührt, mit Essigester extrahiert und der Extrakt über Magnesiumsulfat getrocknet. Es wurde zum Teil im Vakuum eingeengt und dann über Kieselgel mit Ether/Petrolether 1:5 fraktioniert chromatographiert. Die ersten Fraktionen enthielten die Titelverbindung vom Fp. 57-61^{o}C in einer Ausbeute von 12,5 g (58,5 %).

### Beispiel II.5 2-Amino-4-trifluormethoxy-6-trifluormethyl-pyrimidin

4,7 g (0,278 mol) gasförmiges Ammoniak wurden unter Rühren bei -75 bis -70°C innerhalb 1 Stunde in eine Mischung von 38,0 g (0,147 mol) 2-Fluor(chlor)-4-trifluormethoxy-6-trifluormethyl-pyrimidin in 150 ml Diethylether eingegast. Es wurde jeweils 2 Stunden bei -75 und nach dem Erwärmen bei 25^{o}C gerührt. Nach dem Absaugen von dem aufgefallenen Niederschlag wurde die organische Phase mit Wasser 3x extrahiert, getrocknet und teilweise eingeengt. Nach dem Chromatographieren mit Methyl-tert.-butylether über Kieselgel erhielt man 20,4 g (56,1 % d. Th.) der Titelverbindung vom Fp. 47-49^{o}C.

### III Umsetzung der Pyrimidine I zu herbiziden Sulfonylharnstoffderivaten

### Beispiel III.1 bis III.8

### Beispiel III.1 2-Amino-4-methoxy-6-trifluormethoxy-pyrimidin

2,7 g (0,015 mol) 30%iges Natriummethylat wurden innerhalb 15 Minuten unter Rühren bei -5 bis 0^{o}C zu 2,95 g (0,015 mol) 2-Amino-4-fluor-6-trifluormethoxy-pyrimidin in 50 ml Methanol gegeben. Nach 1 Stunde Rühren bei 0^{o}C und Erwärmen auf 25^{o}C wurde das Reaktionsgemisch im Vakuum eingeengt, mit Wasser verrührt und 2x mit Methylenchlorid extrahiert. Nach dem Trocknen und Einengen im Vakuum erhielt man 3,1 g (98 % d. Th.) der Titelverbindung mit n_{D}²⁵ = 1,4770.

### Beispiel III.2 2-Amino-4-chlordifluormethoxy-6-methoxy-pyrimidin

26,1 g (0, 145 mol) 30%iges Natriummethylat wurden innerhalb 15 Minuten unter Rühren bei -10 bis 0^{o}C zu 31,0 g (0,145 mol) 2-Amino-4-chlordifluormethoxy-6-fluorpyrimidin in 300 ml Methanol gegeben. Es wurde 30 Minuten bei 0^{o}C und 1 Stunde bei 25^{o}C gerührt. Das Reaktionsgemisch wurde im Vakuum eingeengt und wie oben aufgearbeitet. Man erhielt 31,6 g (96,6 % d. Th.) der Titelverbindung als farbloses Öl mit n_{D}²² = 1,5039.

### Beispiel III.3 4-Chlordifluormethoxy-2-methylamino-6-methoxy-pyrimidin

4,7 g (0,026 mol) 30%iges Natriummethylat wurden innerhalb 10 Minuten unter Rühren bei 0^{o}C zu 6,0 g (0,0263 mol) 4-Chlordifluormethoxy-6-fluor-2-methylamino-pyrimidin in 100 ml Methanol gegeben. Es wurde jeweils 1 Stunde bei 0^{o}C und bei 25^{o}C gerührt. Nach üblicher Aufarbeitung erhielt man 6,3 g (100 % d. Th.) der Titelverbindung vom Fp. 49-53^{o}C.

### Beispiel III.4 4-Chlordifluormethoxy-6-dimethylamino-2-methylamino-pyrimidin

1,9 g (0,0417 mol) gasförmiges Dimethylamin wurden innerhalb 10 Minuten unter Rühren bei 0°C in eine Mischung von 8,9 g (0,0417 mol) 2-Amino-4-chlordifluormethoxy-6-fluor-pyrimidin in 100 ml Tetrahydrofuran eingeleitet. Es wurde 1 Stunde bei 0°C und 2 Stunden bei 25°C gerührt. Nach üblicher Aufarbeitung erhielt man 9,7 g (97,5 % d. Th.) der Titelverbindung vom Fp. 127-130°C.

### Beispiel III.5 2-(((4-fluor-6-trifluormethoxy-1,3-pyrimidin-2-yl)aminocarbonyl)-amino-sulfonyl)-benzoesäuremethylester

3,6 g (0,015 mol) 2-Isocyanatosulfonyl-benzoesäuremethylester in 15 ml 1,2-Dichlorethan wurden innerhalb 15 Minuten bei 25°C unter Rühren zu einer Mischung von 2,95 g (0,015 mol) 2-Amino-4-fluor-6-trifluormethoxy-pyrimidin in 100 ml 1,2-Dichlorethan gegeben und 12 Stunden bei 25°C gerührt. Die Reaktionslösung wurde im Vakuum eingeengt und der Rückstand mit Ether/Petrolether 1:1 verrührt. Nach dem Absaugen und Trocknen erhielt man 4,8 g (73,3 % d. Th.) der Titelverbindung vom Fp. 157-161°C.

### Beispiel III.6 2-(((4-Chlor-6-trifluormethoxy-1,3-pyrimidin-2-yl)aminocarbonyl)-amino-sulfonyl)-benzoesäure-ethylester

2,55 g (0,01 mol) 2-Isocyanatosulfonyl-benzoesäure-ethylester in 10 ml Methylenchlorid wurden innerhalb 10 Minuten unter Rühren bei 25°C zu einer Mischung von 2,1 g (0,01 mol) 2-Amino-4-chlor-6-trifluormethoxy-pyrimidin in 100 ml Methylenchlorid gegeben. Es wurde 12 Stunden bei 25°C gerührt und von wenig Unlöslichem abgesaugt. Nach dem Einengen des Filtrats im Vakuum, Verrühren des Rückstandes mit Ether/Petrolether 1:1, Absaugen und Trocknen erhielt man 4,0 g (85,4 % d. Th.) der Titelverbindung vom Fp. 148-151°C.

### Beispiel III.7 2-(((4-Methoxy-6-trifluormethoxy-1,3-pyrimidin-2-yl)aminocarbonyl)-aminosulfonyl)-benzoesäuremethylester

4,8 g (0,02 mol) 2-Isocyanatosulfonyl-benzoesäuremethylester in 10 ml Acetonitril wurden innerhalb 15 Minuten unter Rühren bei 25°C zu einer Mischung von 4,1 g (0,02 mol) 2-Amino-4-methoxy-6-trifluormethoxy-pyrimidin in 100 ml Acetonitril gegeben und 12 Stunden nachgerührt. Nach dem Abtrennen des ausgefallenen Niederschlages (2,4 g vom Fp. 141-143^{o}C) wurde das Filtrat im Vakuum eingeengt und mit Ether/Petrolether verrührt, abgesaugt und getrocknet. Man erhielt weitere 4,3 g der Titelverbindung vom Fp. 141-143^{o}C. Die Gesamtausbeute betrug 6,7 g (74,4 % d. Th.).

### Beispiel III.8 2-(((4-Methoxy-6-trifluormethoxy-1, 3-pyrimidin-2-yl)aminocarbonyl)-aminosulfonyl)-benzoesäuremethylester-natriumsalz

2,4 g (0,053 mol) 2-(((4-Methoxy-6-trifluormethoxy-1,3-pyrimidin-2-yl) -aminocarbonyl)aminosulfonyl)-benzoesäuremethylester (Wirkstoffbeispiel 5.001) wurden in 50 ml Methanol gelöst, bei 25°C mit 1,0 g (0,053 mol) 30%iger Natriummethylatlösung in Methanol versetzt und 10 Minuten gerührt. Nach dem Abdestillieren des Lösungsmittels bei vermindertem Druck erhielt man 2,5 g (100 % d. Th.) der Titelverbindung vom Fp. 175^{o}C.

## Patentansprüche

1. Substituierte 2-Amino-(fluoralkoxy-pyrimidine) der allgemeinen Formel I, 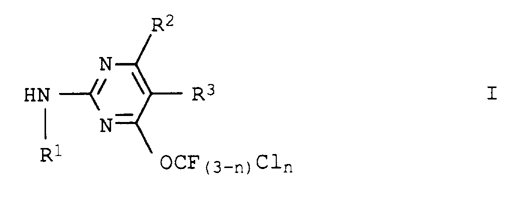 in der
R¹ Wasserstoff, C₁-C₄-Alkyl, C₃-C₄-Alkenyl oder C₃-C₄-Alkinyl
R² Wasserstoff, Halogen oder C₁-C₄-Halogenalkyl sowie auch einen Trifluor- bzw. Chlordifluormethoxyrest
R³ Wasserstoff und
n 0 oder 1
bedeuten.

2. Verfahren zur Herstellung von 2-Amino-(fluoralkoxy-pyrimidinen) der allgemeinen Formel I, 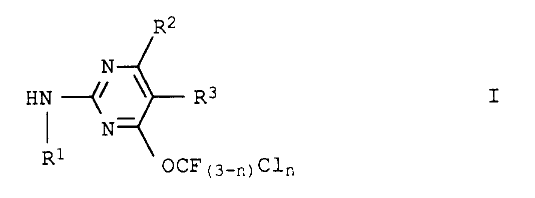 in der
R¹ Wasserstoff, C₁-C₄-Alkyl, C₃-C₄-Alkenyl oder C₃-C₄-Alkinyl
R² Wasserstoff, Halogen oder C₁-C₄-Halogenalkyl,
R³ Wasserstoff und
n 0 oder 1
bedeuten,
dadurch gekennzeichnet, daß man 2-Halogen-pyrimidine der Formel II, 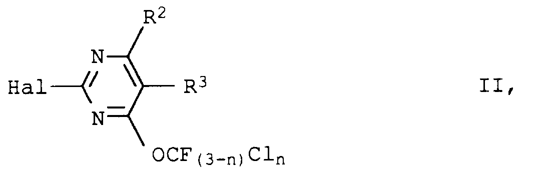 in der Hal für Fluor oder Chlor steht und R², R³ und n die vorgenannte Bedeutung besitzen, mit einem Amin der Formel III,
H-NH-R¹ III,
in der R¹ die vorgenannte Bedeutung hat, ggf. in Gegenwart einer organischen Hilfsbase, umsetzt.

3. Verfahren nach Anspruch 2, dadurch gekennzeichnet, daß man als Amin Ammoniak verwendet.

4. Verfahren nach Anspruch 2, dadurch gekennzeichnet, daß man als Amin Methylamin einsetzt.

5. Verfahren nach den Ansprüchen 2 und 4, dadurch gekennzeichnet, daß man die Umsetzung bei einer Temperatur von -80 bis 40°C durchführt.

6. 2-Amino-4-fluor-6-trifluormethoxy-pyrimidin.

7. 2-Amino-4-chlor-6-trifluormethoxy-pyrimidin.

8. 2-Amino-4-chlordifluormethoxy-6-fluor-pyrimidin.

9. 2-Amino-4-chlor-6-chlordifluormethoxy-pyrimidin.

## Claims

1. A substituted 2-amino(fluoroalkoxy)pyrimidine of the formula I 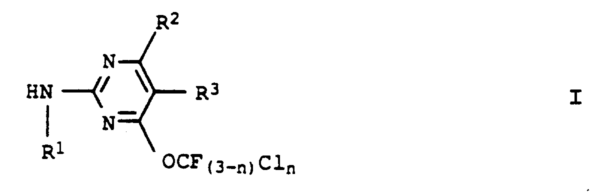 where
R¹ is hydrogen, C₁-C₄-alkyl, C₃-C₄-alkenyl or C₃-C₄-alkynyl,
R² is hydrogen, halogen or C₁-C₄-haloalkyl, or else trifluoromethoxy or chlorodifluoromethoxy,
R³ is hydrogen and
n is 0 or 1.

2. A process for preparing a 2-amino(fluoroalkoxy)pyrimidine of the formula I 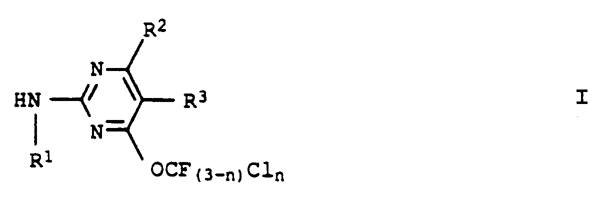 where
R¹ is hydrogen, C₁-C₄-alkyl, C₃-C₄-alkenyl or C₃-C₄-alkynyl,
R² is hydrogen, halogen or C₁-C₄-haloalkyl,
R³ is hydrogen and
n is 0 or 1,
which comprises reacting a 2-halopyrimidine of the formula II 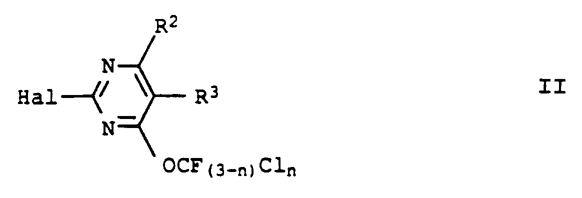 where Hal is fluorine or chlorine, and R², R³ and n have the abovementioned meanings, with an amine of the formula III
H-NH-R¹ III
where R¹ has the abovementioned meaning, in the presence or absence of an organic base.

3. A process as claimed in claim 2, wherein ammonia is used as amine.

4. A process as claimed in claim 2, wherein methyl-amine is employed as amine.

5. A process as claimed in either of claims 2 and 4, wherein the reaction is carried out at from -80 to 40°C.

6. 2-Amino-4-fluoro-6-trifluoromethoxypyrimidine.

7. 2-Amino-4-chloro-6-trifluoromethoxypyrimidine.

8. 2-Amino-4-chlorodifluoromethoxy-6-fluoro-pyrimidine.

9. 2-Amino-4-chloro-6-chlorodifluoromethoxy-pyrimidine.

## Revendications

1. 2-amino-(fluoralcoxy-pyrimidines) substituées de formule générale I 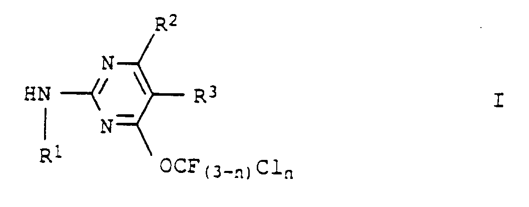 dans laquelle
R¹ représente hydrogène, alkyle en C1-C4, alcényle en C3-C4 ou alcynyle en C3-C4,
R² représente hydrogène, halogène ou halogénalkyle en C1-C4 ainsi qu'un reste trifluoro- ou chlorodifluorométhoxy,
R³ représente hydrogène et
n est 0 ou 1.

2. Procédé de préparation de 2-amino-(fluoralcoxy-pyrimidines) de formule générale I 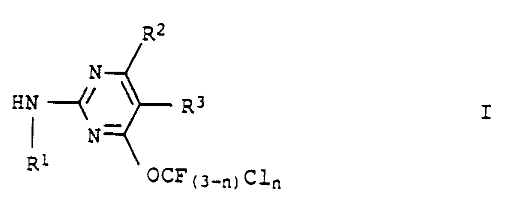 dans laquelle
R¹ représente hydrogène, alkyle en C1-C4, alcényle en C3-C4 ou alcynyle en C3-C4,
R² représente hydrogène, halogène ou halogénalkyle en C1-C4,
R³ représente hydrogène et
n est 0 ou 1,
caractérisé par le fait qu'on fait réagir, éventuellement en présence d'une base auxiliaire organique, des 2-halogéno-pyrimidines de formule II 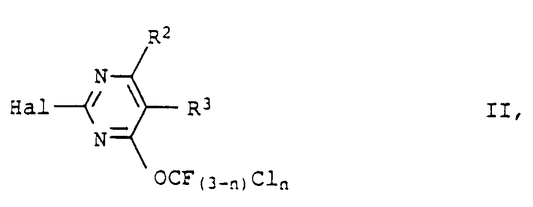 dans laquelle Hal est mis pour fluor ou chlore et R², R³ et n ont la signification sus-indiquée, avec une amine de la formule III
H-NH-R¹ III
dans laquelle R¹ a la signification sus-indiquée.

3. Procédé selon la revendication 2, caractérisé par le fait qu'on utilise de l'ammoniac en tant qu'amine.

4. Procédé selon la revendication 2, caractérisé par le fait qu'on introduit de la méthylamine en tant qu'amine.

5. Procédé selon les revendications 2 et 4, caractérisé par le fait qu'on effectue la réaction à une température de -80 à 40°C.

6. 2-amino-4-fluoro-6-trifluorométhoxy-pyrimidine.

7. 2-amino-4-chloro-6-trifluorométhoxy-pyrimidine.

8. 2-amino-4-chlorodifluorométhoxy-6-fluoro-pyrimidine.

9. 2-amino-4-chloro-6-chlorodifluorométhoxy-pyrimidine.
